# EUROPEAN PATENT APPLICATION

(11) **EP 3 851 064 A1**
(43) Date of publication of application: **21.07.2021**
(21) Application number: 21151171.2
(22) Date of filing: 12.01.2021
(51) Int. Cl.: A61B 90/57, A61B 17/072

(54) **HANDHELD ELECTROMECHANICAL SURGICAL INSTRUMENTS**

(30) Priority: 14.01.2020 US 202062960751 P; 14.12.2020 US 202017120840
(71) Applicant: Covidien LP, Mansfield, MA 02048 (US)
(72) Inventor: RAMADAN, Ryanne, Northborough, Massachusetts 01532 (US); EBNER, Timothy, Southington, Connecticut 06489 (US)
(74) Representative: Maschio, Antonio

(57) **Abstract**

A surgical handle assembly includes a power pack, an outer shell housing configured to selectively encase the power pack therein, and a sensor assembly attached to the outer shell housing. The sensor assembly includes a plurality of sensors for gathering and storing information about a surgical procedure.

## Description

### CROSS-REFERENCE TO RELATED APPLICATIONS

This application claims the benefit of and priority to U.S. Provisional Patent Application Serial No. 62/960,751 filed January 14, 2020, the entire disclosure of which is incorporated by reference herein.

### BACKGROUND

### 1. Technical Field

This disclosure relates to surgical instruments. More specifically, this disclosure relates to outer shell housings of electromechanical surgical instruments with sensing capabilities.

### 2. Background of Related Art

One type of surgical instrument is a linear clamping, cutting and stapling instrument. Such an instrument may be employed in a surgical procedure to resect a cancerous or anomalous tissue from a gastro-intestinal tract. Conventional linear clamping, cutting and stapling instruments include a pistol grip-styled structure having an elongated shaft and distal portion. The distal portion includes a pair of scissors-styled gripping elements, which clamp the open ends of the colon closed. In this instrument, one of the two scissors-styled gripping elements, such as the anvil portion, moves or pivots relative to the overall structure, whereas the other gripping element remains fixed relative to the overall structure. The actuation of this scissoring mechanism (the pivoting of the anvil portion) is controlled by a grip trigger maintained in the handle.

In addition to the gripping elements, the distal portion also includes a stapling mechanism. The fixed gripping element of the scissoring mechanism includes a staple cartridge receiving region and a mechanism for driving the staples up through the clamped end of the tissue against the anvil portion, thereby sealing the previously opened end. The gripping elements may be integrally formed with the shaft or may be detachable such that various scissoring and stapling elements may be interchangeable.

A number of surgical instrument manufacturers have developed product lines with proprietary powered drive systems for operating and/or manipulating the surgical instrument. In many instances the surgical instruments include a powered handle assembly, which is reusable, and a disposable end effector or the like that is selectively connected to the powered handle assembly prior to use and then disconnected from the end effector following use in order to be disposed of or in some instances sterilized for re-use.

### SUMMARY

In one aspect of the disclosure, a surgical handle assembly is provided and includes a power pack, an outer shell housing configured to selectively encase the power pack therein, and a sensor assembly. The power pack includes a motor and a drive shaft coupled to and rotatable by the motor. The sensor assembly is coupled to the outer shell housing.

In aspects, the sensor assembly may include a sensor such as an accelerometer, a temperature sensor, a strain gauge, a magnetometer, or a gyroscope.

In some aspects, the sensor may be configured to be in communication with the power pack to transfer sensed information to the power pack.

In other aspects, the sensor assembly may include a sensor housing configured to be coupled to the outer shell housing, and a sensor disposed within the sensor housing.

In further aspects, the outer shell housing may include a proximal portion and a distal portion pivotably connected to the proximal portion. The proximal portion may have an upper shell portion, and the sensor housing may be configured to couple to the upper shell portion.

In aspects, the sensor housing may be configured to clip onto the upper shell portion.

In some aspects, the sensor housing may have a body portion in which the at least one sensor is housed, and a pair of curved, flexible arms extending from the body portion. The flexible arms may be configured to detachably clip onto the upper shell portion.

In further aspects, sensor assembly may include a battery supported in the sensor housing and in electrical communication with the sensor.

In other aspects, the sensor assembly may include an inductive coupling supported in the sensor housing, and the power pack may have an inductive coupling configured to be inductively coupled to the inductive coupling of the sensor assembly.

In aspects, the power pack may include a processor in electrical communication with the inductive coupling of the power pack, such that sensed information from the sensor may be passed from the sensor to the processor.

In some aspects, the sensor assembly may include a printed circuit board supported in the sensor housing. The printed circuit board may have a processor and a memory in communication with the sensor for storing the sensed information.

In accordance with an aspect of the disclosure, a surgical handle assembly is provided and includes a power pack, an outer shell housing configured to selectively encase the power pack therein, and a sensor assembly. The power pack includes a motor and a drive shaft coupled to and rotatable by the motor. The sensor assembly is disposed within the outer shell housing and includes a sensor.

In aspects, the sensor assembly may include a flexible printed circuit board having the sensor attached thereto.

In some aspects, the flexible printed circuit board may have a proximal end portion attached to a proximal portion of the outer shell housing, and a distal end portion attached to a distal portion of the outer shell housing.

In further aspects, the proximal and distal portions of the outer shell housing may be pivotably coupled to one another, such that the outer shell housing is transitionable between an open and closed configuration.

In other aspects, the power pack may include an electrical connector and a processor in electrical connection with the electrical connector. The distal end portion of the flexible printed circuit board may have an electrical connector configured to connect with the electrical connector of the power pack.

In aspects, the flexible circuit board may have a processor and a memory. The memory may be in communication with the sensor for storing the sensed information.

In some aspects, the sensor may be an accelerometer, a temperature sensor, a strain gauge, a magnetometer, and/or a gyroscope.

In further aspects, the sensor may be configured to be in communication with the power pack to transfer sensed information to the power pack.

In other aspects, the sensor assembly may include an inductive coupling, and the power pack may have an inductive coupling configured to be inductively coupled to the inductive coupling of the sensor assembly.

In aspects, the power pack may include a processor in electrical communication with the inductive coupling of the power pack, such that sensed information from the sensor is passed from the sensor to the processor.

In accordance with yet another aspect of the disclosure, an outer shell housing for selectively encasing a power pack therein is provided. The outer shell housing includes a proximal portion defining a cavity therein, a distal portion defining a cavity therein and pivotably coupled to the proximal portion, and a sensor assembly. The proximal and distal portions are configured to move between an open configuration, in which a portion of the proximal portion is spaced from a corresponding portion of the distal portion, and a closed configuration, in which the portion of the proximal portion is connected to the corresponding portion of the distal portion. The sensor assembly is configured to be coupled to one or both of the proximal portion or the distal portion. The sensor assembly includes a printed circuit board having a sensor, a processor, and a memory in communication with the sensor for storing information sensed by the sensor.

In aspects, the sensor may be an accelerometer, a temperature sensor, a strain gauge, a magnetometer, and/or a gyroscope.

In some aspects, the sensor assembly may include a sensor housing configured to be coupled to one or both of the proximal portion or the distal portion. The printed circuit board may be supported in the sensor housing.

In further aspects, the proximal portion may have an upper shell portion, and the sensor housing of the sensor assembly may be configured to couple to the upper shell portion.

In other aspects, the sensor housing may be configured to clip onto the upper shell portion.

In aspects, the sensor housing may have a body portion in which the sensor is housed, and a pair of curved, flexible arms extending from the body portion. The flexible arms may be configured to detachably clip onto the upper shell portion.

In some aspects, the sensor assembly may include a battery supported in the sensor housing and in electrical communication with the sensor.

In further aspects, in the closed configuration, the proximal portion and the distal portion may cooperatively define an internal cavity configured for encasing a power pack, and in the open configuration, a power pack is insertable or removable from the outer shell housing.

In accordance with a further aspect of the disclosure, an outer shell housing for selectively encasing a power pack therein is provided. The outer shell housing includes a proximal portion defining a cavity therein, a distal portion defining a cavity therein and pivotably coupled to the proximal portion, and a sensor assembly. The proximal and distal portions are configured to move between an open configuration, in which a portion of the proximal portion is spaced from a corresponding portion of the distal portion, and a closed configuration, in which the portion of the proximal portion is connected to the corresponding portion of the distal portion. The sensor assembly is disposed within the outer shell housing and includes a flexible printed circuit board having a sensor attached thereto.

In aspects, the flexible printed circuit board may include a proximal end portion and a distal end portion. The proximal end portion may be attached to an inner surface of the proximal portion of the outer shell housing, and the distal end portion may be attached to an inner surface of the distal portion of the outer shell housing.

In some aspects, flexible circuit board may have a processor and a memory. The memory may be in communication with the sensor for storing information sensed by the sensor.

In further aspects, the sensor may be an accelerometer, a temperature sensor, a strain gauge, a magnetometer, and/or a gyroscope.

### BRIEF DESCRIPTION OF THE DRAWINGS

Embodiments of the present disclosure are described herein with reference to the accompanying drawings, wherein:
FIG. 1 is a perspective view of a handheld surgical instrument including a handle assembly, an adapter assembly, and a surgical loading unit, in accordance with an embodiment of the disclosure;
FIG. 2 is a front perspective view of the handle assembly of FIG. 1;
FIG. 3 is a front perspective view, with parts separated, of the handle assembly of FIG. 2 including an outer shell housing and a power pack;
FIG. 4A is a side view of the surgical instrument of FIG. 1 illustrating the outer shell housing of FIG. 3 in an open configuration
FIG. 4B is a side view of the surgical instrument of FIG. 1 illustrating the outer shell housing of FIG. 3 in the open configuration with the power pack disposed therein;
FIG. 4C is a side view of the surgical instrument of FIG. 1 illustrating the outer shell housing of FIG. 3 in a closed configuration;
FIG. 5 is a perspective view illustrating the outer shell housing of FIG. 3 and a sensor assembly clipped thereto;
FIG. 6 is a side, perspective view illustrating the handle assembly of FIG. 1 including the sensory assembly of FIG. 5, with an outer housing portion removed;
FIG. 7 is a schematic diagram illustrating a circuit of the sensor assembly of FIG. 5;
FIG. 8 is a top view of a plurality of sensors of the circuit of FIG. 7;
FIG. 9 is a perspective view illustrating another embodiment of a handle assembly including a power pack, an outer shell housing, and a sensor assembly;
FIG. 10 is a top perspective view illustrating the outer shell housing of FIG. 9 and the sensor assembly disposed therein; and
FIG. 11 is a schematic diagram illustrating a circuit of the sensor assembly of FIG. 9.

### DETAILED DESCRIPTION OF EMBODIMENTS

Embodiments of the presently disclosed surgical instruments including handle assemblies, adapter assemblies, and sensor assemblies, are described in detail with reference to the drawings, in which like reference numerals designate identical or corresponding elements in each of the several views. As used herein the term "distal" refers to that portion of the surgical instrument, or component thereof, farther from the user, while the term "proximal" refers to that portion of the surgical instrument, or component thereof, closer to the user.

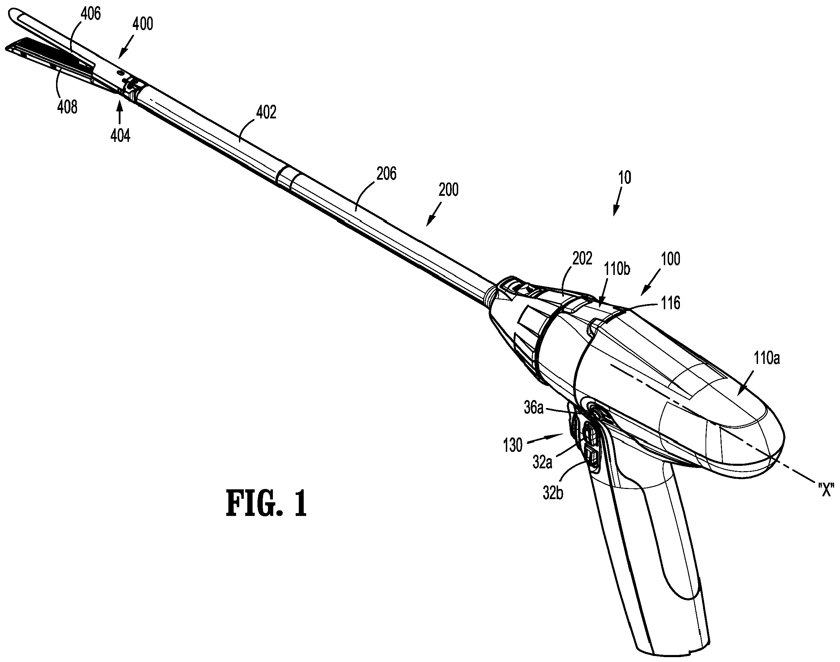

With reference to FIG. 1, a surgical instrument, in accordance with an embodiment of the disclosure, is generally designated as 10, and is in the form of a powered hand held electromechanical instrument configured for performing various surgical functions, for example, stapling and cutting tissue. The surgical instrument 10 includes a handle assembly 100 configured for selective connection with an adapter assembly 200, and, in turn, the adapter assembly 200 is configured for selective connection with end effectors or single use loading units ("SULU's") 400.

As illustrated in FIGS. 1-4C, the handle assembly 100 of the surgical instrument 10 includes an outer shell housing 110 and a power pack 101 configured to be selectively received and substantially encased by the outer shell housing 110. The outer shell housing 110 includes a proximal portion or proximal half-section 110a and a distal portion or distal half-section 110b. The half-sections 110a, 110b of the outer shell housing 110 are pivotably connected to one another by a hinge 116 located along an upper edge of the distal half-section 110b and the proximal half-section 110a. When joined, the proximal and distal half-sections 110a, 110b define a shell cavity 110c therein in which the power-pack 101 is selectively situated. The proximal and distal half-sections 110a, 110b are divided along a plane that is perpendicular to a longitudinal axis "X" of the adapter assembly 200. Each of the proximal and distal half-sections 110a, 110b includes a respective upper shell portion 112a, 112b, and a respective lower shell portion 114a, 114b. The lower shell portions 112a, 112b define a snap closure feature 118 for selectively securing the lower shell portions 112a, 112b to one another and for maintaining the outer shell housing 110 in a closed condition.

The proximal half-section 110a is sized and shaped to house a majority of the power pack 101 therein. The proximal half-section 110a of the shell housing 110 supports a right-side control button 36a and a left-side control button 36b. The right-side control button 36a and the left-side control button 36b are capable of being actuated upon application of a corresponding force thereto or a depressive force thereto.

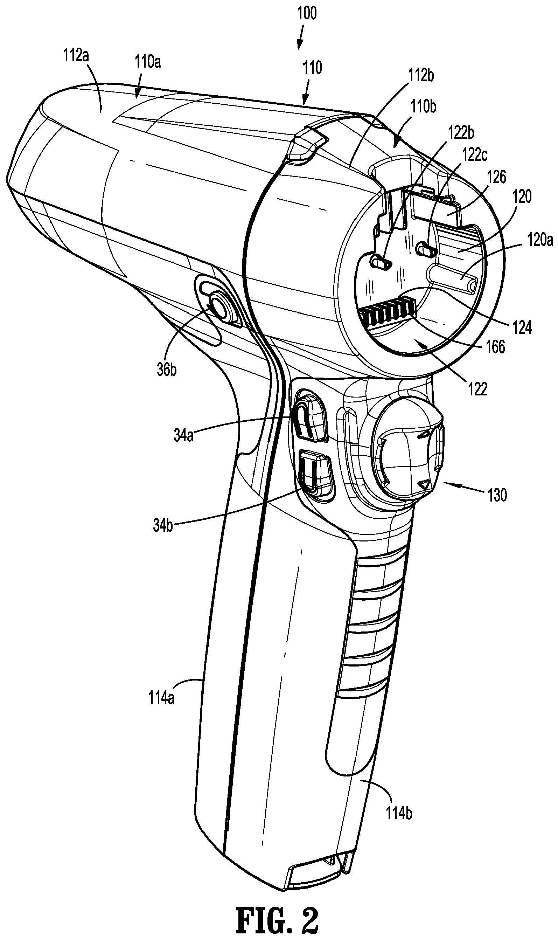

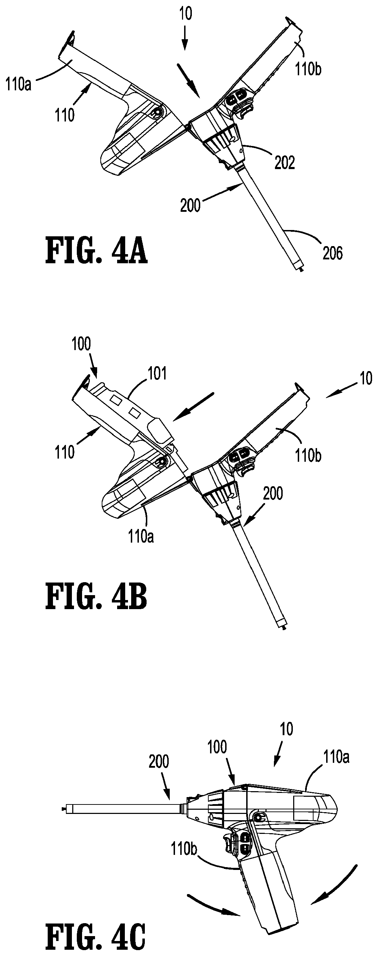

The distal half-section 110b of the outer shell housing 110 covers a distal facing portion of the power pack 101 when the outer shell housing 110 is in the closed configuration, as shown in FIGS. 2 and 4C. The distal half-section 110b defines a connecting portion 120 configured to accept a corresponding drive coupling assembly (not shown) of the adapter assembly 200. Specifically, the distal half-section 110b of the outer shell housing 110 defines a recess 122 that receives a portion (not shown) of the drive coupling assembly (not shown) of the adapter assembly 200 when the adapter assembly 200 is mated to the handle assembly 100. The connecting portion 120 of the distal half-section 110b defines a pair of axially extending guide rails 120a, 120b projecting radially inward from inner side surfaces thereof. The guide rails 120a, 120b assist in rotationally orienting the adapter assembly 200 relative to the handle assembly 100 when the adapter assembly 200 is mated to the handle assembly 100.

The connecting portion 120 of the distal half-section 110b defines three apertures 122a, 122b, 122c formed in a distally facing surface thereof and which are arranged in a common plane or line with one another. The connecting portion 120 of the distal half-section 110b also defines an elongate slot 124 through which an electrical pass-through connector 166 extends. The connecting portion 120 of the distal half-section 110b further defines a female connecting feature 126 formed in a surface thereof. The female connecting feature 126 selectively engages with a male connecting feature (not shown) of the adapter assembly 200.

The distal half-section 110b of the outer shell housing 110 supports a distal facing toggle control button 130. The toggle control button 130 is capable of being actuated in a left, right, up, and down direction upon application of a corresponding force thereto or a depressive force thereto. The distal half-section 110b of the outer shell housing 110 supports a right-side pair of control buttons 32a, 32b; and a left-side pair of control button 34a, 34b. The right-side control buttons 32a, 32b and the left-side control buttons 34a, 34b are capable of being actuated upon application of a corresponding force thereto or a depressive force thereto.

The outer shell housing 110 is fabricated from a polycarbonate or similar polymer, and is clear or transparent and/or may be overmolded. In some embodiments, the outer shell housing 110 may be fabricated from any suitable material that can be sterilized, for example, by way of autoclaving. The outer shell housing 110 may be provided as a sterilized unit to the clinician or surgeon, for receipt of the power-pack 101 (which may or may not be sterile).

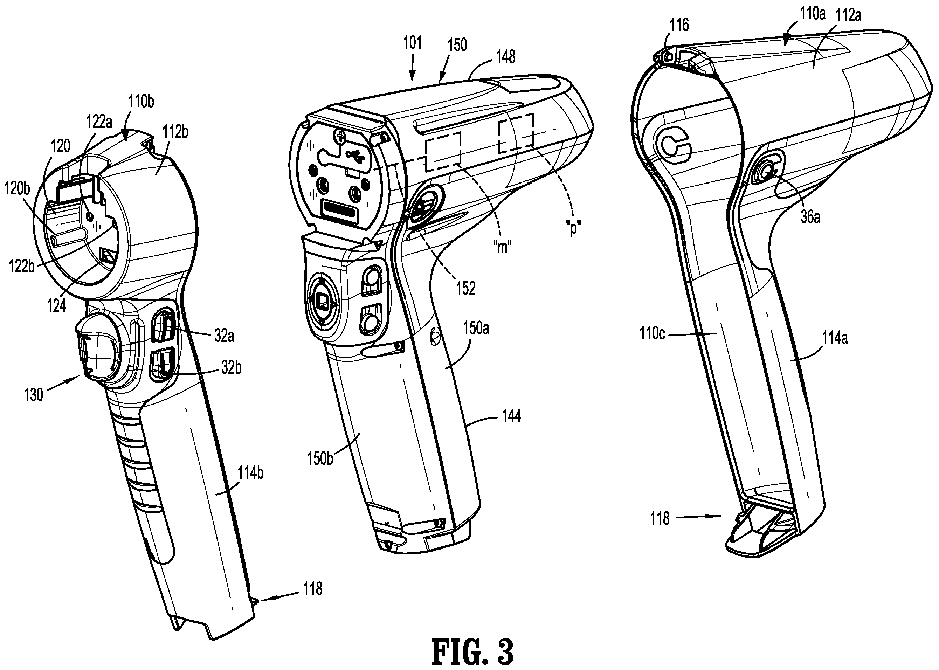

With reference to FIGS. 3-4C, the power-pack 101 of the handle assembly 100 is configured for receipt within the outer shell housing 110 and for powering the functions of the surgical instrument 10. The power-pack 101 of the handle assembly 100 includes an inner handle housing 150 having a lower housing portion 144 and an upper housing portion 148 extending from and/or supported on the lower housing portion 144. The lower housing portion 144 and the upper housing portion 148 are separated into a proximal half-section 150a and a distal half-section 150b connectable to the proximal half-section 150a by a plurality of fasteners. When joined, the proximal and distal half-sections 150a, 150b define an inner handle housing 150 having an inner housing cavity (not shown) therein in which a power-pack core assembly (not shown) is situated. The power-pack core assembly is configured to control the various operations of the surgical instrument 10.

The inner handle housing 150 of the power pack 101 provides a housing in which the power-pack core assembly is situated. The power-pack core assembly includes a battery circuit (not shown), a controller circuit board or processor "P" (FIG. 3) and a rechargeable battery (not shown) configured to supply power to any of the electrical components of the handle assembly 100. The processor "P" includes a motor controller circuit board (not shown), a main controller circuit board (not shown), and a first ribbon cable (not shown) interconnecting the motor controller circuit board and the main controller circuit board.

The inner handle housing 150 further includes a memory having stored therein instructions or programs to be executed by the processor "P." The memory is configured to store data regarding the operation of the surgical instrument 10 that may be accessed later by a clinician or an engineer. The memory may include an RFID, flash memory EEPROM, EPROM, or any suitable non-transitory storage chip that stores information about the operation of the surgical instrument 10.

The power-pack core assembly further includes a motor "M" electrically connected to the controller circuit board and the battery. It is contemplated that the power-pack core assembly may include more than one motor, for example, a second motor (not shown) and a third motor (not shown). The motor "M" is disposed between the motor controller circuit board and the main controller circuit board. The power-pack core assembly has a motor shaft or a drive shaft 152 coupled to and rotatable by the motor "M."

The motor "M" is controlled by a motor controller. The motor controller is disposed on the motor controller circuit board and is, for example, A3930/31K motor drivers from Allegro Microsystems, Inc. The A3930/31K motor drivers are designed to control a 3-phase brushless DC (BLDC) motor with N-channel external power MOSFETs, such as the motor "M". Each of the motor controllers is coupled to a main controller disposed on the main controller circuit board. The main controller is also coupled to the memory, which is also disposed on the main controller circuit board. The main controller is, for example, an ARM Cortex M4 processor from Freescale Semiconductor, Inc, which includes 1024 kilobytes of internal flash memory. The main controller communicates with the motor controllers through an FPGA, which provides control logic signals (e.g., coast, brake, etc.). The control logic of the motor controller then outputs corresponding energization signals to the motor "M" using fixed-frequency pulse width modulation (PWM).

Rotation of the motor shafts 152 by the motors "M" of the power pack 101 function to drive shafts and/or gear components of the adapter assembly 200 in order to perform the various operations of the surgical instrument 10. For example, the motor "M" of the power-pack 101 may be configured to drive shafts and/or gear components of the outer shell housing 110, which drive corresponding driven shafts and/or gear components of the adapter assembly 200 in order to selectively move a tool assembly 404 (FIG. 1) of the SULU 400 relative to a proximal body portion 402 of the SULU 400, to rotate the SULU 400 about a longitudinal axis "X," to move a cartridge assembly 408 relative to an anvil assembly 406 of the SULU 400, and/or to fire staples from within the cartridge assembly 408 of the SULU 400.

The adapter assembly 200 includes an outer knob housing 202 and an outer tube 206 extending from a distal end of the knob housing 202. The knob housing 202 and the outer tube 206 are configured and dimensioned to house the components of the adapter assembly 200. The outer tube 206 is dimensioned for endoscopic insertion. In particular, the outer tube 206 is passable through a typical trocar port, cannula or the like. The knob housing 202 is dimensioned to not enter the trocar port, cannula or the like. The knob housing 202 is configured to connect to the connecting portion 120 of the outer shell housing 110 of the handle assembly 100.

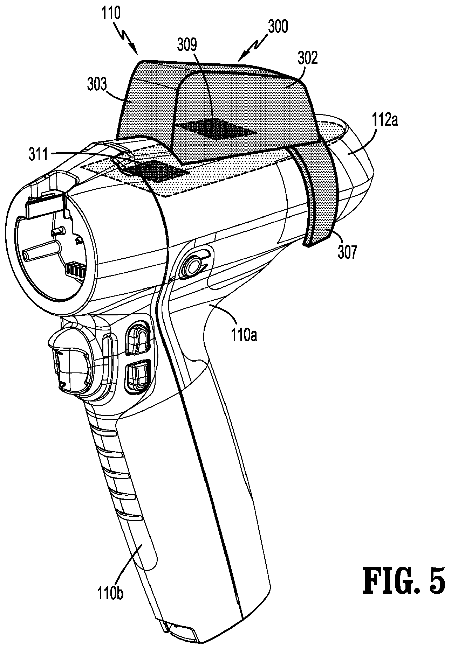

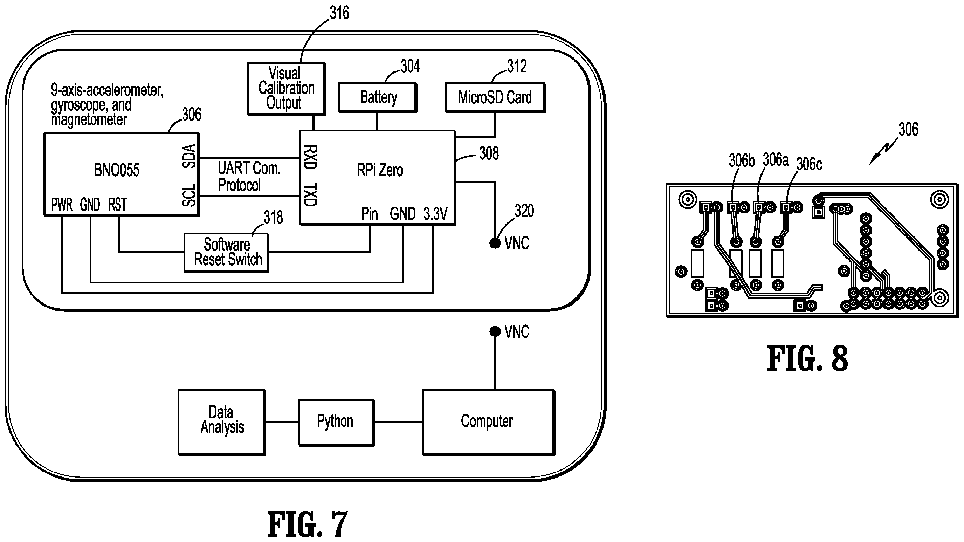

With reference to FIGS. 5-8, the outer shell housing 110 further includes a sensor assembly 300 attached/attachable to an outer surface thereof. For example, the sensor assembly 300 may be detachably or permanently coupled to the upper shell portion 112a of the proximal half-section 110a. In aspects, the sensor assembly 300 may detachably connected to any suitable portion of the outer shell housing 110 via any suitable fastening mechanism, such as, for example, hook and loop fasteners, adhesives, a bayonet-type connection, or the like. It is contemplated that the sensor assembly 300 may be configured as a clip that may be mechanically detachable from the outer shell housing 110.

The sensor assembly 300 includes a housing 302, a battery 304 supported in the housing 302, and one or more sensors 306 electrically coupled to the battery 304. The housing 302 has a body portion 303 in which the sensors 306 are housed, and a pair of curved, flexible arms 307 extending from the body portion 303. The sensor assembly 300 may include an inductive coupling 309, and the power pack 101 may include an inductive coupling 311, such that signals may be received between the processor "P" (FIG. 3) of the power pack 101 and a processor 310 of the sensor assembly 300. In some aspects, the sensor assembly 300 may inductively receive power from the power pack 101.

The sensors 306 may include an accelerometer 306a (e.g., a 9-axis accelerometer), a magnetometer 306b, and/or a gyroscope 306c, cooperatively configured to determine an orientation of the handle assembly 100 (e.g., pitch, roll, yaw, etc.). In some aspects, the sensors 306 may be configured to determine the orientation of the handle assembly 100 in all six degrees of freedom. The sensor assembly 300 may additionally or alternatively include speakers and microphones (not explicitly shown) for aiding a clinician in troubleshooting, acoustic emission and vibration sensors to detect gear box failure, and ultra wide band positioning system for providing surgical feedback and ergonomic input.

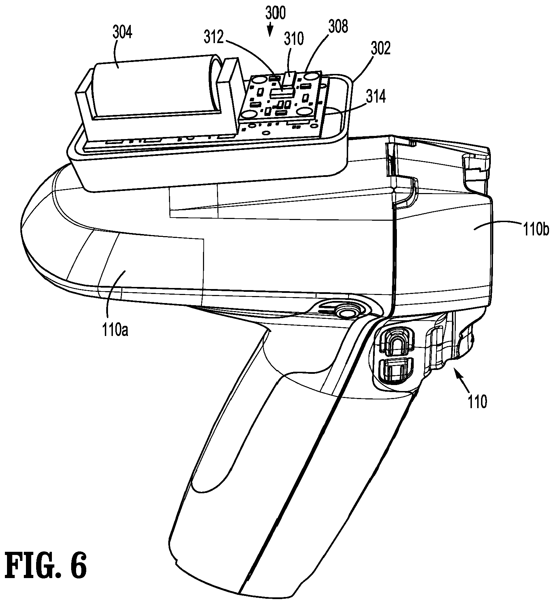

With reference to FIGS. 6-8, the sensor assembly 300 may include a system on a chip 308 that integrates the sensors 306, the processor 310 (e.g., a central processing unit), and a memory 312 all supported on a printed circuit board 314. The system on a chip 308 is received within the housing 302 and is in electrical communication with the battery 304. The system on a chip 308 may be in wireless communication (e.g., Bluetooth, near-field communication etc.) with the processor "P" (FIG. 1) of the power pack 101, such that information sensed by the sensors 306 may be communicated to the processor "P" and/or memory of the power pack 101 for storage therein. The system on a chip 308 may be electrically connected to a visual calibration output 316, a software reset switch 318, and a visual network computing 320.

Information gathered by the sensor assembly 300 may be directly overlaid with data logged from the surgical instrument 10 to provide complete resolution of the procedure to aid in design development and improve surgical efficiency. For example, the data gathered by the sensor assembly 300 may include time to completion of a distinct surgical step in a procedure, motion smoothness, response orientation, articulation angle, number of firings, sizes of surgical loading units 400, and battery usage, which may be utilized by a clinician in real time or after a procedure to improve their skills and ultimately improve patient outcomes. Other feedback data provided by the sensor assembly 300 may include perfusion measurements, clamp duration, tissue force, tissue temperature, and foreign object detection. The sensor assembly 300 may wirelessly communicate the data to the memory within the surgical instrument 10 or to a computer located remotely from the surgical instrument 10, such as a phone application.

In aspects, the sensor assembly 300 may include various other sensors 306 configured to measure light level, temperature, force/pressure, position, speed, and/or sound. For example, the sensor assembly 300 may include Light Dependant Resistors (LDR), photodiodes, phototransistors, solar cells, thermocouples, thermistors, thermostats, resistive temperature detectors, strain gauges, pressure switches, load cells, potentiometers, encoders, reflective/slotted optocouplers, Doppler Effect sensors, carbon microphones, piezo-electrical crystals, etc., and/or combinations thereof.

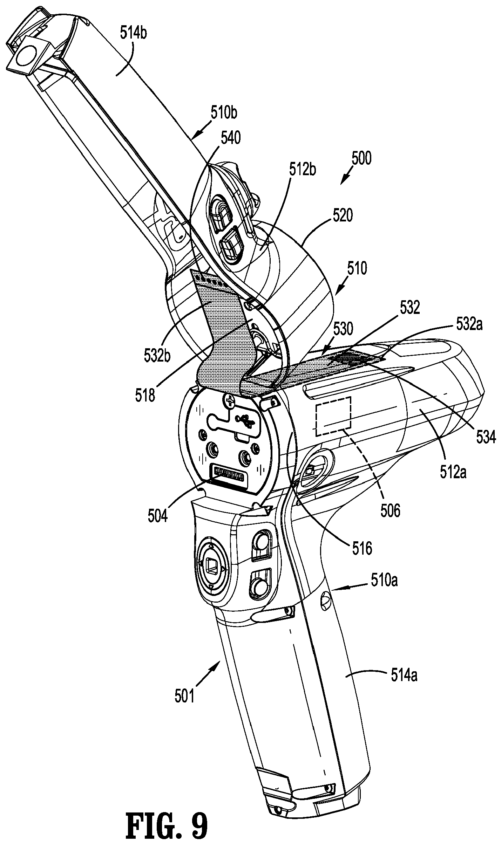

FIGS. 9-11 illustrate another embodiment of a handle assembly 500, similar to the handle assembly 100 described above. The handle assembly 500 is different by having the sensor assembly 530 disposed within the outer shell housing 510. Due to the similarities between the two handle assemblies, only those elements of the handle assembly 500 deemed necessary to elucidate the differences from the handle assembly 100 will be described in detail.

The handle assembly 500 includes an outer shell housing 510 and a power pack 501 configured to be selectively received and substantially encased by the outer shell housing 510. The outer shell housing 510 includes a proximal portion or proximal half-section 510a and a distal portion or distal half-section 510b. The half-sections 510a, 510b of the outer shell housing 510 are pivotably connected. Each of the proximal and distal half-sections 510a, 510b includes a respective upper shell portion 512a, 512b, and a respective lower shell portion 514a, 514b. The distal half-section 510b defines a connecting portion 520 configured to accept a corresponding drive coupling assembly (not shown) of the adapter assembly 200 (FIG. 1)

The outer shell housing 510 further includes a sensor assembly 530 disposed therein. The sensor assembly 530 includes a flexible printed circuit board 532 having a plurality of sensors 534 attached thereto. The flexible printed circuit board 532 may be elongated and include a proximal end portion 532a received in the proximal portion 510a of the outer shell housing 510, and a distal end portion 532b received in the distal portion 510b of the outer shell housing 510. In particular, the proximal end portion 532a of the flexible circuit board 532 may be attached, via adhesive, to an inner surface 516 of the upper shell portion 512a of the proximal portion 510a of the outer shell housing 510. In aspects, the proximal end portion 532a of the flexible circuit board 532 may be attached to the proximal portion 510a via any suitable fastening engagement. The distal end portion 532b of the flexible circuit board 532 is attached to an inner surface 518 of the upper shell portion 512b of the distal portion 510b of the outer shell housing 510.

The distal end portion 532b of the flexible circuit board 532 may have an electrical connector 540, such as, for example, a pin adapter, received in an elongate slot (not explicitly shown) in the distal portion 510b of the outer shell portion 510. The electrical connector 540 is configured to engage an electrical connector 504, such as, for example, an electrical receptacle of the power pack 501 when the outer shell housing 510 is in the closed configuration. The power pack 501 includes a processor 506 in electrical connection with the electrical connector 504 thereof for receiving sensed information from the sensor assembly 530 via the electrical connector 540.

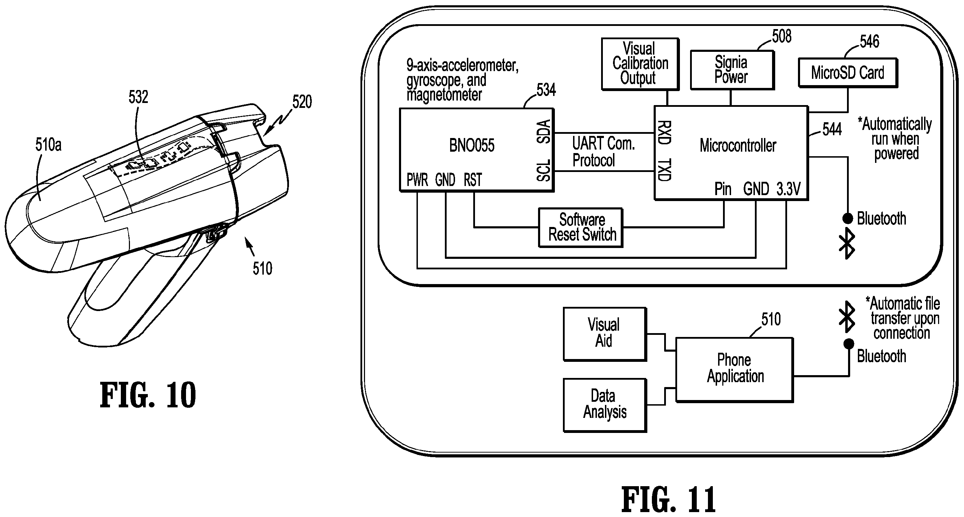

With reference to FIG. 11, the flexible circuit board 532 has a processor 544, such as, for example, a microcontroller, and a memory 546 in communication with the sensors 534 for storing the sensed information. The sensors 534 are configured to be in communication with the power pack 501 to transfer sensed information to the power pack 501. The processor 544 of the sensor assembly 530 is in electrical communication with a battery 508 of the power pack 501 and/or the processor 506 of the power pack 501, such that the sensor assembly 530 is powered by the battery 508 of the power pack 501 and sensed information from the sensors 534 may be passed from the sensors 534 to the processor 506 and/or memory of the power pack 501. The processor 544 of the sensor assembly 530 may be configured to wirelessly connect (e.g., via Bluetooth, near-field communication, etc.) with an external device 510 (e.g., a phone application) to send sensed information from the sensors 534 to the external device.

It will be understood that various modifications may be made to the embodiments of the presently disclosed adapter assemblies. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A surgical handle assembly, comprising: a power pack including: a motor; and a drive shaft coupled to and rotatable by the motor; an outer shell housing configured to selectively encase the power pack therein; and a sensor assembly coupled to the outer shell housing.
2. The surgical handle assembly according to claim 1, wherein the sensor assembly includes at least one sensor selected from the group consisting of an accelerometer, a temperature sensor, a strain gauge, a magnetometer, and a gyroscope.
3. The surgical handle assembly according to claim 2, wherein the at least one sensor is configured to be in communication with the power pack to transfer sensed information to the power pack.
4. The surgical handle assembly according to claim 1, wherein the sensor assembly includes: a sensor housing configured to be coupled to the outer shell housing; and at least one sensor disposed within the sensor housing.
5. The surgical handle assembly according to claim 4, wherein the outer shell housing includes:
   a proximal portion having an upper shell portion, the sensor housing configured to couple to the upper shell portion; and a distal portion pivotably connected to the proximal portion.
6. The surgical handle assembly according to claim 5, wherein the sensor housing of the sensor assembly is configured to clip onto the upper shell portion.
7. The surgical handle assembly according to claim 5, wherein the sensor housing has a body portion in which the at least one sensor is housed, and a pair of curved, flexible arms extending from the body portion and configured to detachably clip onto the upper shell portion.
8. The surgical handle assembly according to claim 4, wherein the sensor assembly includes a battery supported in the sensor housing and in electrical communication with the at least one sensor.
9. The surgical handle assembly according to claim 4, wherein the sensor assembly includes an inductive coupling supported in the sensor housing, and the power pack has an inductive coupling configured to be inductively coupled to the inductive coupling of the sensor assembly.
10. The surgical handle assembly according to claim 9, wherein the power pack includes a processor in electrical communication with the inductive coupling of the power pack, such that sensed information from the at least one sensor is passed from the at least one sensor to the processor.
11. The surgical handle assembly according to claim 4, wherein the sensor assembly includes a printed circuit board supported in the sensor housing, the printed circuit board having a processor and a memory in communication with the at least one sensor for storing the sensed information.
12. A surgical handle assembly, comprising: a power pack including: a motor; and a drive shaft coupled to and rotatable by the motor; an outer shell housing configured to selectively encase the power pack therein; and a sensor assembly disposed within the outer shell housing and including at least one sensor.
13. The surgical handle assembly according to claim 12, wherein the sensor assembly includes a flexible printed circuit board having the at least one sensor attached thereto.
14. The surgical handle assembly according to claim 13, wherein the flexible printed circuit board has: a proximal end portion attached to a proximal portion of the outer shell housing; and a distal end portion attached to a distal portion of the outer shell housing.
15. The surgical handle assembly according to claim 14, wherein the proximal and distal portions of the outer shell housing are pivotably coupled to one another, such that the outer shell housing is transitionable between an open and closed configuration.
16. The surgical handle assembly according to claim 15, wherein the power pack includes: an electrical connector; and a processor in electrical connection with the electrical connector, the distal end portion of the flexible printed circuit board having an electrical connector configured to connect with the electrical connector of the power pack.
17. The surgical handle assembly according to claim 13, wherein the flexible circuit board has a processor and a memory, the memory being in communication with the at least one sensor for storing the sensed information.
18. The surgical handle assembly according to claim 12, wherein the at least one sensor is selected from the group consisting of an accelerometer, a temperature sensor, a strain gauge, a magnetometer, and a gyroscope.
19. The surgical handle assembly according to claim 12, wherein the at least one sensor is configured to be in communication with the power pack to transfer sensed information to the power pack.
20. The surgical handle assembly according to claim 12, wherein the sensor assembly includes an inductive coupling, and the power pack has an inductive coupling configured to be inductively coupled to the inductive coupling of the sensor assembly.

## Claims

1. A surgical handle assembly, comprising:
a power pack including:
a motor; and
a drive shaft coupled to and rotatable by the motor;
an outer shell housing configured to selectively encase the power pack therein; and
a sensor assembly coupled to the outer shell housing.

2. The surgical handle assembly according to claim 1, wherein the sensor assembly includes at least one sensor selected from the group consisting of an accelerometer, a temperature sensor, a strain gauge, a magnetometer, and a gyroscope.

3. The surgical handle assembly according to claim 2, wherein the at least one sensor is configured to be in communication with the power pack to transfer sensed information to the power pack.

4. The surgical handle assembly according to any preceding claim, wherein the sensor assembly includes:
a sensor housing configured to be coupled to the outer shell housing; and
at least one sensor disposed within the sensor housing.

5. The surgical handle assembly according to claim 4, wherein the outer shell housing includes:
a proximal portion having an upper shell portion, the sensor housing configured to couple to the upper shell portion; and
a distal portion pivotably connected to the proximal portion.

6. The surgical handle assembly according to claim 5, wherein the sensor housing of the sensor assembly is configured to clip onto the upper shell portion; and/or wherein the sensor housing has a body portion in which the at least one sensor is housed, and a pair of curved, flexible arms extending from the body portion and configured to detachably clip onto the upper shell portion.

7. The surgical handle assembly according to claim 4, wherein the sensor assembly includes a battery supported in the sensor housing and in electrical communication with the at least one sensor; and/or wherein the sensor assembly includes an inductive coupling supported in the sensor housing, and the power pack has an inductive coupling configured to be inductively coupled to the inductive coupling of the sensor assembly.

8. The surgical handle assembly according to claim 7, wherein the power pack includes a processor in electrical communication with the inductive coupling of the power pack, such that sensed information from the at least one sensor is passed from the at least one sensor to the processor.

9. The surgical handle assembly according to claim 4, wherein the sensor assembly includes a printed circuit board supported in the sensor housing, the printed circuit board having a processor and a memory in communication with the at least one sensor for storing the sensed information.

10. A surgical handle assembly, comprising:
a power pack including:
a motor; and
a drive shaft coupled to and rotatable by the motor;
an outer shell housing configured to selectively encase the power pack therein; and
a sensor assembly disposed within the outer shell housing and including at least one sensor.

11. The surgical handle assembly according to claim 10, wherein the sensor assembly includes a flexible printed circuit board having the at least one sensor attached thereto' preferably wherein the flexible printed circuit board has:
a proximal end portion attached to a proximal portion of the outer shell housing; and
a distal end portion attached to a distal portion of the outer shell housing.

12. The surgical handle assembly according to claim 10 or claim 11, wherein the proximal and distal portions of the outer shell housing are pivotably coupled to one another, such that the outer shell housing is transitionable between an open and closed configuration; preferably wherein the power pack includes:
an electrical connector; and
a processor in electrical connection with the electrical connector, the distal end portion of the flexible printed circuit board having an electrical connector configured to connect with the electrical connector of the power pack.

13. The surgical handle assembly according to any of claims 10 to 12, wherein the flexible circuit board has a processor and a memory, the memory being in communication with the at least one sensor for storing the sensed information.

14. The surgical handle assembly according to any of claims 10 to 13, wherein the at least one sensor is selected from the group consisting of an accelerometer, a temperature sensor, a strain gauge, a magnetometer, and a gyroscope.

15. The surgical handle assembly according to any of claims 10 to 14, wherein the at least one sensor is configured to be in communication with the power pack to transfer sensed information to the power pack; and/or wherein the sensor assembly includes an inductive coupling, and the power pack has an inductive coupling configured to be inductively coupled to the inductive coupling of the sensor assembly.
